(19) Europäisches Patentamt
European Patent Office
Office européen des brevets

(11) EP 0 998 910 B1

(12) EUROPÄISCHE PATENTSCHRIFT

(45) Veröffentlichungstag und Bekanntmachung des Hinweises auf die Patenterteilung:
**16.06.2004 Patentblatt 2004/25**

(51) Int Cl.⁷: **A61K 7/42**

(21) Anmeldenummer: **99119165.1**

(22) Anmeldetag: **06.10.1999**

(54) **Kosmetische und dermatologische Hydrodispersionen mit einem Gehalt an Triglyceridwachsen**

Cosmetic and dermatologic aqueous dispersions containing triglyceridic waxes

Dispersions aqueuses cosmetiques et dermatologiques contenant des cires triglyceridiques

(84) Benannte Vertragsstaaten:
**AT BE CH CY DE DK ES FI FR GB GR IE IT LI LU MC NL PT SE**

(30) Priorität: **10.10.1998 DE 19846772**

(43) Veröffentlichungstag der Anmeldung:
**10.05.2000 Patentblatt 2000/19**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **Knüppel, Anja
20257 Hamburg (DE)**
- **Müller, Anja
23843 Rümpel (DE)**
- **Gronau, Annette
22763 Hamburg (DE)**
- **Dörschner, Albrecht
20146 Hamburg (DE)**

(56) Entgegenhaltungen:
**EP-A- 0 786 246**
**EP-A- 0 787 483**
**WO-A-00/07547**
**WO-A-98/42300**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische und dermatologische Hydrodispersionen.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum comeum), den für die Barrierefunktion bedeutenden Teil darstellt. Das heute in der Fachwelt anerkannte Hautmodell von Elias *(P. M. Elias, Structure and Function of the Stratum Corneum Permeability Barrier, Drug Dev. Res.* **13**, 1988, 97-*105*) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Weiterhin war es eine Aufgabe der Erfindung, kosmetische Grundlagen für kosmetische Zubereitungen zu entwickeln, die sich durch gute Hautverträglichkeit auszeichnen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubsubstanzen zur Verfügung zu haben, da auch dessen Strahlen Schäden hervorrufen können. Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut

"altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Eine andere, europaweit etablierte Prüfungsmethode ist der Australische Standard AS/NZS 2604:1997. Dabei wird die Absorption der Zubereitung im UV-A-Bereich gemessen. Um den Standard zu erfüllen, muß die Zubereitung mindestens 90 % der UV-A-Strahlung im Bereich 320 -360 nm absorbieren.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es eine Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Die UV-Strahlung kann aber auch zu photochemischen Reaktionen führen, wobei dann die photochemischen Reaktionsprodukte in den Hautmetabolismus eingreifen. Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Alterdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Bekannte und vorteilhafte Lichtschutzfiltersubstanzen sind Dibenzoylmethanderivate, beispielsweise 5-Isopropyldibenzoylmethan (CAS-Nr. 63250-25-9), welches sich durch die Struktur

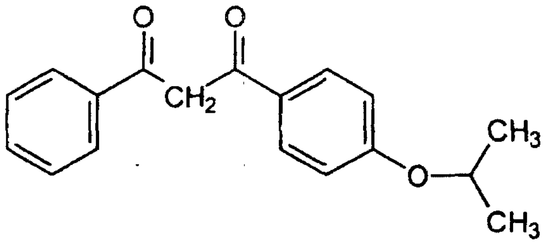

auszeichnet und von Merck unter der Marke Eusolex® 8020 verkauft wird, sowie das 4-(tert.-Butyl)-4'-methoxydibenzoylmethan (CAS-Nr. 70356-09-1), welches sich durch die Struktur

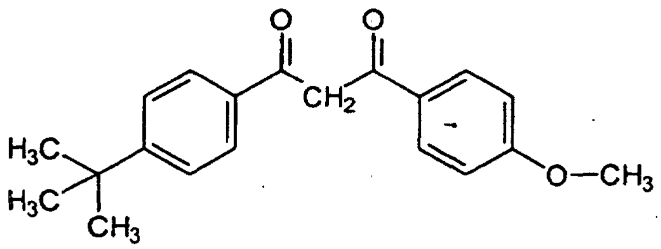

auszeichnet, und von Givaudan unter der Marke Parsol® 1789 verkauft wird. Gerade in Kombination mit anderen als Festkörper vorliegenden Substanzen ist ihre Einsatzkonzentration jedoch begrenzt. Es bereitet daher gewisse formulierungstechnische Schwierigkeiten, höhere Lichtschutzfaktoren zu erzielen.

Eine weitere vorteilhafte Lichtschutzfiltersubstanz ist der 4-Methylbenzylidencampher, welcher sich durch die Struktur

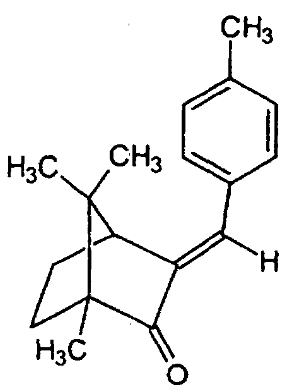

auszeichnet und von Merck unter der Marke Eusolex® 6300 verkauft wird. 4-Methylbenzylidencampher ist eine äußerst vorteilhafte, unter Normalbedingungen als Festkörper vorliegende Lichtschutzfiltersubstanz und zeichnet sich an sich durch gute UV-Filtereigenschaften aus. Gerade in Kombination mit anderen als Festkörper vorliegenden Substanzen ist jedoch auch ihre Einsatzkonzentration jedoch begrenzt. Es bereitet daher auch hier gewisse formulierungstechnische Schwierigkeiten, höhere Lichtschutzfaktoren zu erzielen.

Auch andere Benzylidencampherderivate sind vorteilhafte Lichtschutzfiltersubstanzen, z.B. der Benzylidencampher, welcher sich durch die Struktur

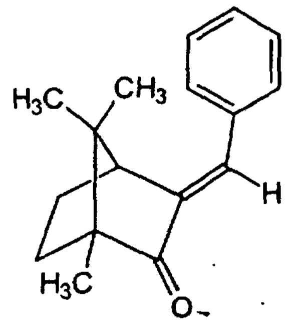

auszeichnet und von der Gesellschaft Induchem unter der Marke Unisol® S22 verkauft wird. Gerade in Kombination mit anderen als Festkörper vorliegenden Substanzen ist jedoch auch ihre Einsatzkonzentration begrenzt. Es bereitet daher auch hier gewisse formulierungstechnische Schwierigkeiten, höhere Lichtschutzfaktoren zu erzielen.

Ein weiterer vorteilhafter UV-Filter ist der 4,4',4"-(1,3,5-Triazin-2,4,6-triyltriimino)-tris-benzoesäure-tris (2-ethylhexylester), synonym: 2,4,6-Tris-[anilino-(p-carbo-2'-ethyl-1'-hexyloxy)]-1,3,5-triazin.

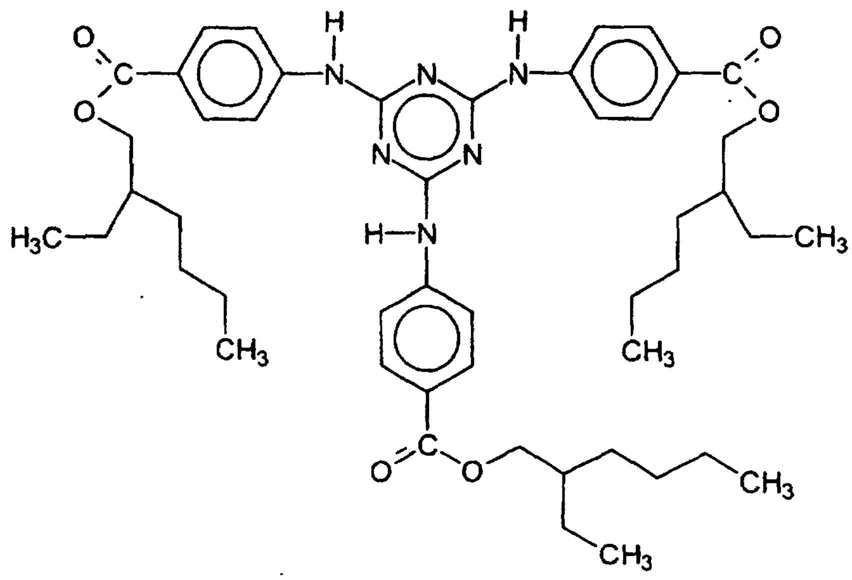

Diese UVB-Filtersubstanz wird von der BASF Aktiengesellschaft unter der Warenbezeichnung UVINUL® T 150 vertrieben und zeichnet sich durch gute UV-Absorptionseigenschaften aus. Zwischenzeitlich wurden von verschiedenen Autoren auch andere UV-Filtersubstanzen beschrieben, welche das Strukturmotiv

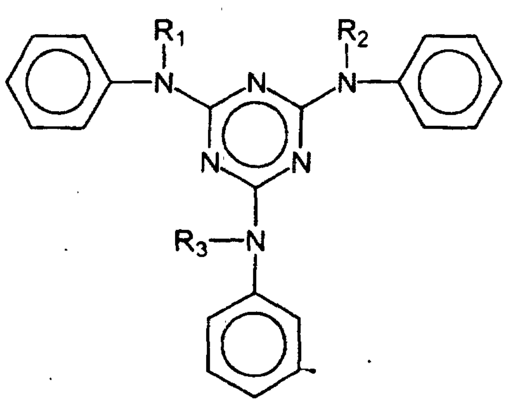

aufweisen.

Der Hauptnachteil des 4,4',4"-(1,3,5-Triazin-2,4,6-triyltriimino)-tris-benzoesäure-tris(2-ethylhexylesters) ist seine schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz. Es bereitet daher auch hier gewisse formulierungstechnische Schwierigkeiten, höhere Lichtschutzfaktoren zu erzielen.

Auch andere als Festkörper vorliegende UV-Filtersubstanzen, deren Einarbeitung in kosmetische oder dermatologische Lichtschutzformulierungen zumindest gewisse Probleme aufweist, sind bekannt. So werden in der EP-A-570 838 s-Triazinderivate beschrieben, deren chemische Struktur durch die generische Formel

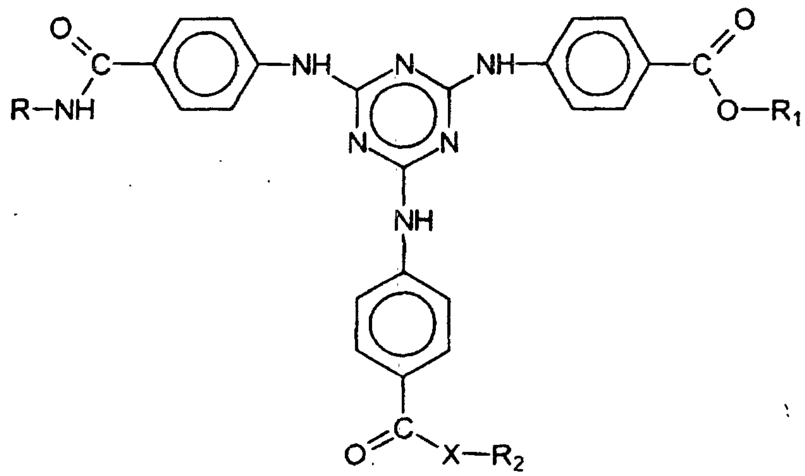

wiedergegeben wird, wobei

- R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,
- X ein Sauerstoffatom oder eine NH-Gruppe darstellt,
- $R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

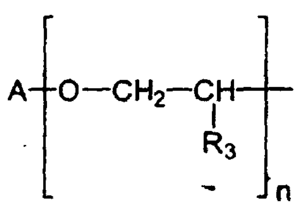

bedeutet, in welcher

- A einen verzweigten oder unverzweigten $C_1$-$C_{18}$-Alkylrest, einen $C_5$-$C_{12}$Cycloalkyl- oder Arylrest darstellt, gegebenenfalls substituiert mit einer oder mehreren $C_1$-$C_4$- Alkylgruppen,
- $R_3$ ein Wasserstoffatom oder eine Methylgruppe darstellt,
- n eine Zahl von 1 bis 10 darstellt,

- $R_2$ einen verzweigten oder unverzweigten $C_1$-$C_{18}$-Alkylrest, einen $C_5$-$C_{12}$-Cyctoalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

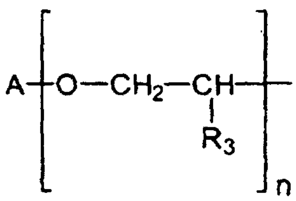

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,

wenn X ein Sauerstoffatom darstellt.

Ein Beispiel für solche unsymmetrisch substituierte s-Triazine stellt das Dioctylbutylamidotriazon dar, dessen chemische Struktur durch die Formel

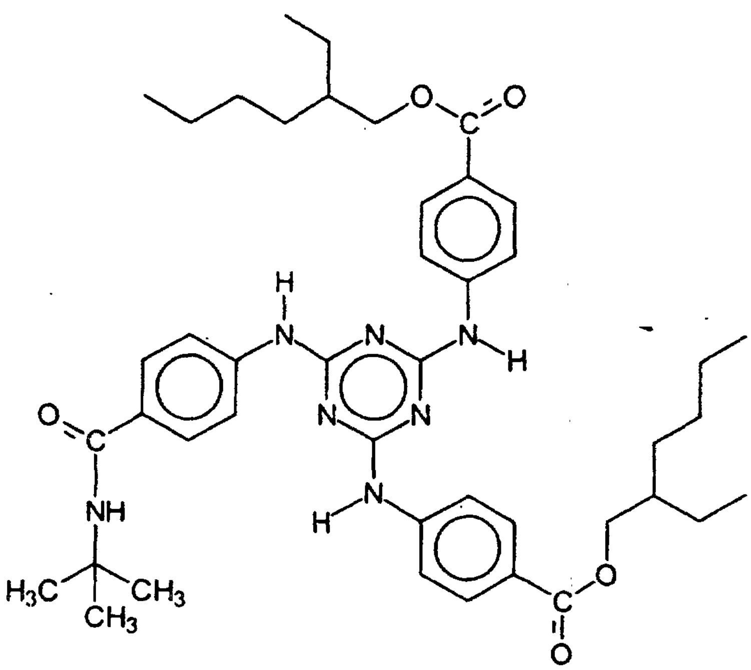

wiedergegeben wird.

Auch andere UV-Filtersubstanzen, deren Einarbeitung in kosmetische oder dermatologische Lichtschutzformulierugen zumindest gewisse Probleme aufweist, sind bekannt. So werden in der EP-A-775 698 Bis-Resorcinyltriazinderivate beschrieben, deren chemische Struktur durch die generische Formel

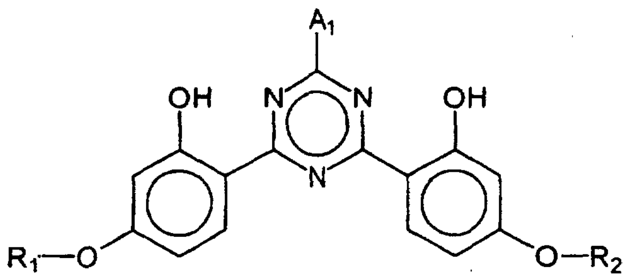

wiedergegeben wird, wobei $R_1$, $R_2$ und $A_1$ verschiedenste organische Reste repräsentieren.

Vorteilhafte Bis-Resorcinyltriazinderivate sind ferner beispielsweise folgende Verbindungen:

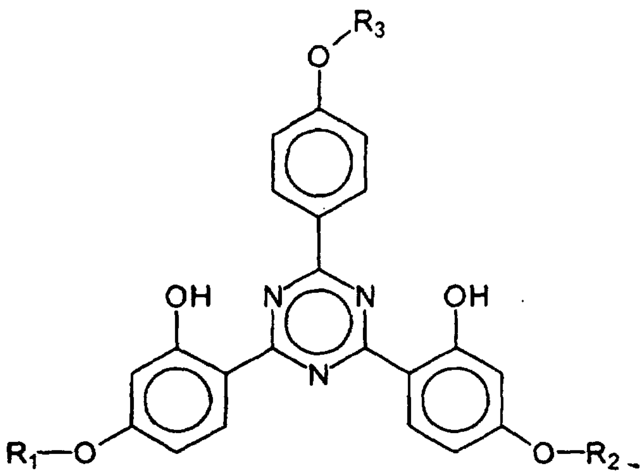

wobei $R_3$ ein Wasserstoffatom oder eine verzweigte oder unverzweigte Alkylgruppe mit 1 bis 10 Kohlenstoffatomen darstellt und $R_1$ und $R_2$ die vorgenannten Bedeutungen haben, insbesondere das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

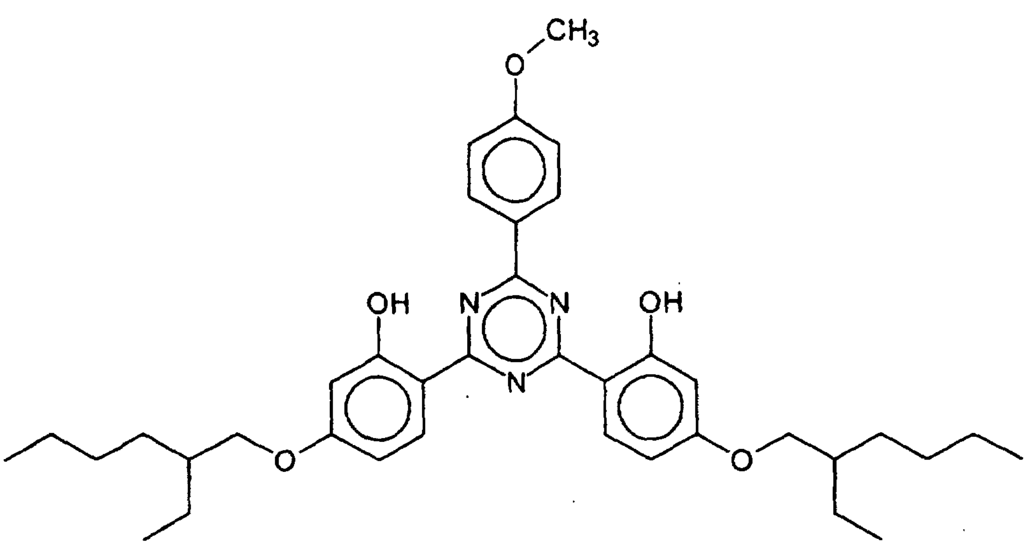

Ferner vorteilhaft ist das 2,4-Bis-{[4-(3-sulfonato)-2-hydroxy-propyloxy)-2-hydroxy]-phenyl}-6-(4-methoxyphenyl)-1,3,5-triazin Natriumsalz, welches durch folgende Struktur gekennzeichnet ist:

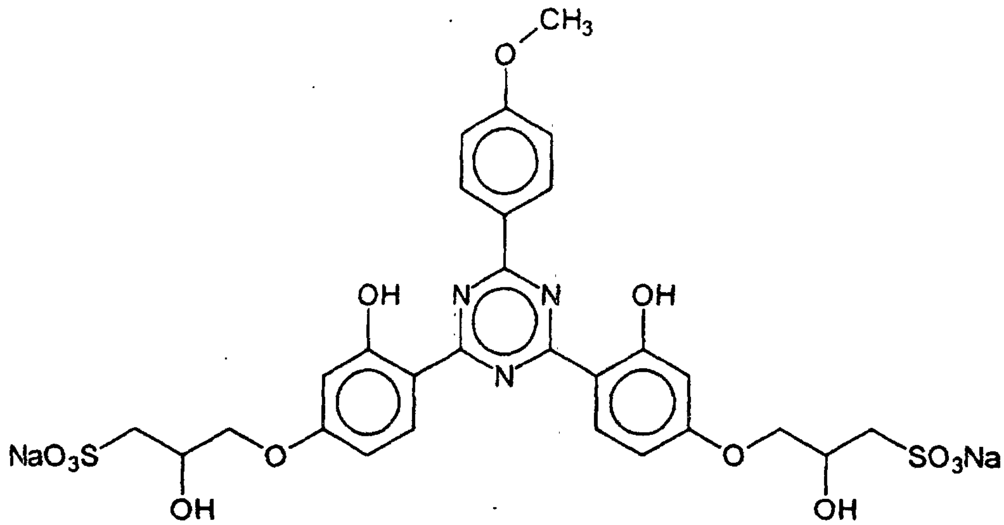

Ferner vorteilhaft ist das 2,4-Bis-{[4-(3-(2-Prolpyloxy)-2-hydroxy-propyloxy)-2-hydroxy]-phenyl}-6-(4-methoxyphenyl)-1,3,5-triazin, welches durch folgende Struktur gekennzeichnet ist:

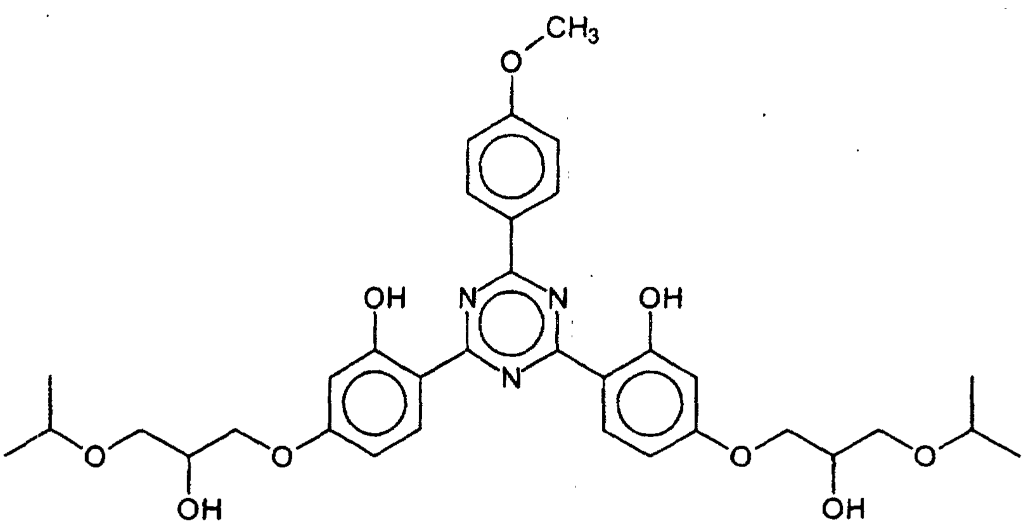

Ferner vorteilhaft ist das 2,4-Bis-{[4-(2-ethyl-hexyloxy)-2-hydroxy]-phenyl}-6-[4-(2-methoxyethyl-carboxyl}-phenylamino}-1,3,5-triazin, welches durch folgende Struktur gekennzeichnet ist:

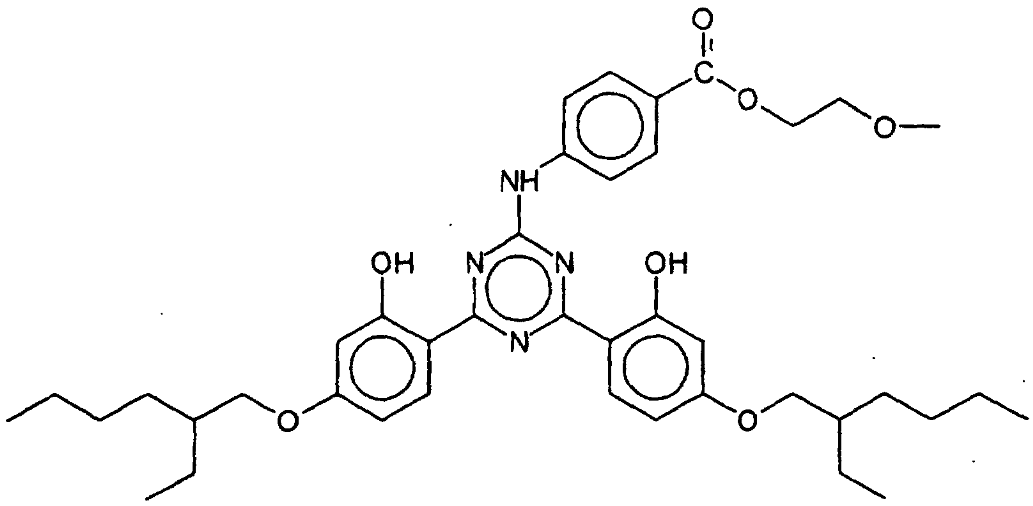

Ferner vorteilhaft ist das 2,4-Bis-([4-(3-(2-propyloxy)-2-hydroxy-propyloxy)-2-hydroxy]-phenyl}-6-[4-(2-ethylcarboxyl)-phenylamino]-1,3,5-triazin, welches durch folgende Struktur gekennzeichnet ist:

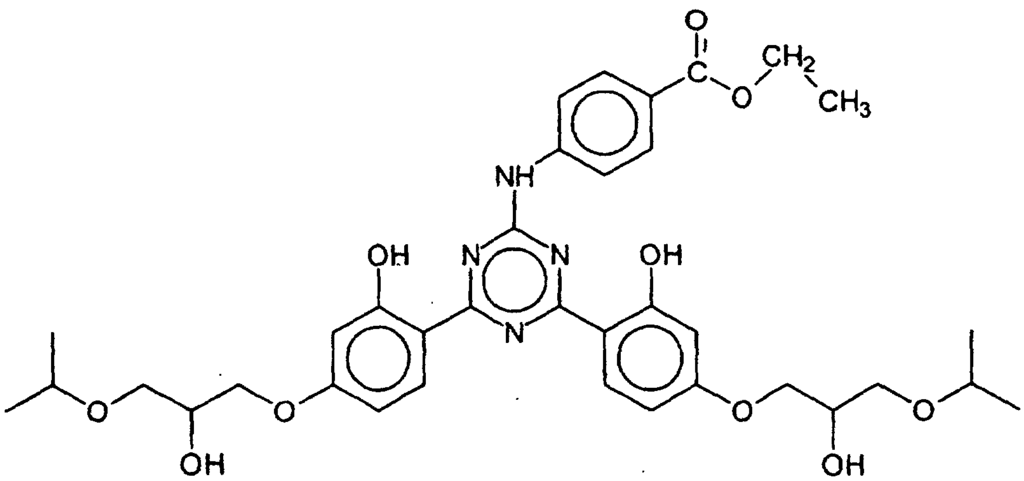

Ferner vorteilhaft ist das 2,4-Bis-{[4-(2-ethyl-hexyloxy)-2-hydroxy]-phenyl}-6-(1-methyl-pyrrol-2-yl)-1,3,5-triazin, welches durch folgende Struktur gekennzeichnet ist:

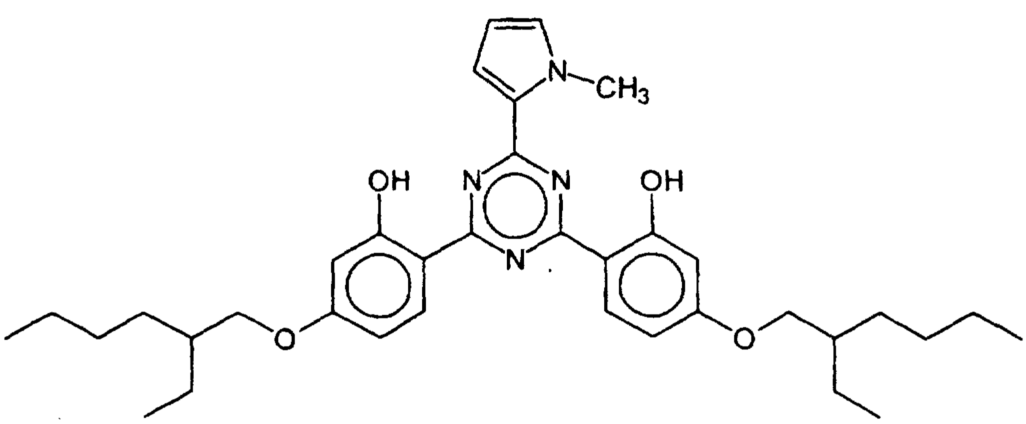

Ferner vorteilhaft ist das 2,4-Bis-{(4-tris(trimethylsiloxy-silylpropyloxy)-2-hydroxy]-phenyl}-6-(4-methoxyphenyl)-1,3,5-triazin, welches durch folgende Struktur gekennzeichnet ist:

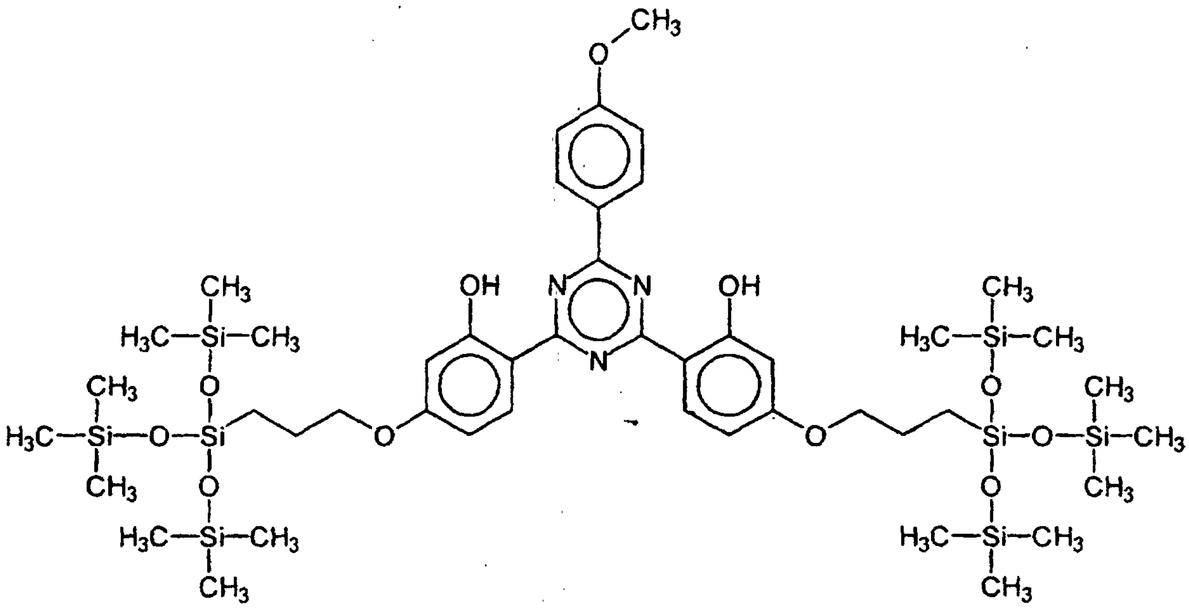

Ferner vorteilhaft ist das 2,4-Bis-{[4-(2''-methylpropenyloxy}-2-hydroxy]-phenyl}-6-{4-methoxyphenyl)-1,3,5-triazin, welches durch folgende Struktur gekennzeichnet ist:

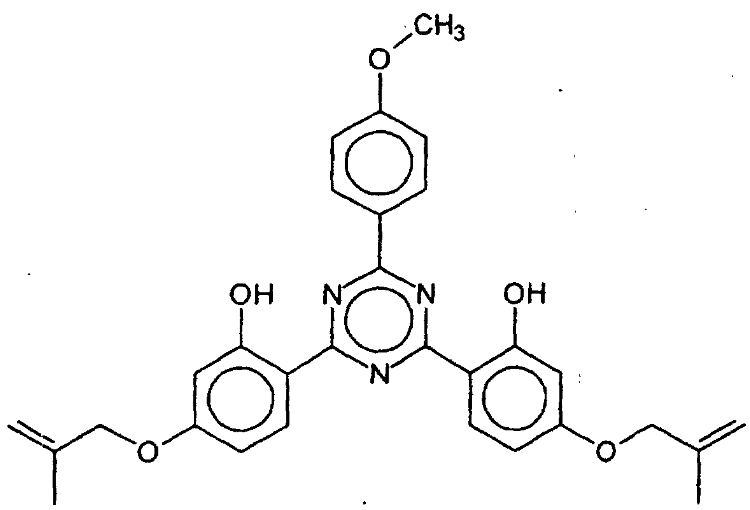

Ferner vorteilhaft ist das 2,4-Bis-{[4-(1',1',1',3',5',5',5'-Heptamethylsiloxy-2''-methyl-propyloxy)-2-hydroxy]-phenyl}-6-(4-methoxyphenyi)-1,3,5-triazin, welches durch folgende Struktur gekennzeichnet ist:

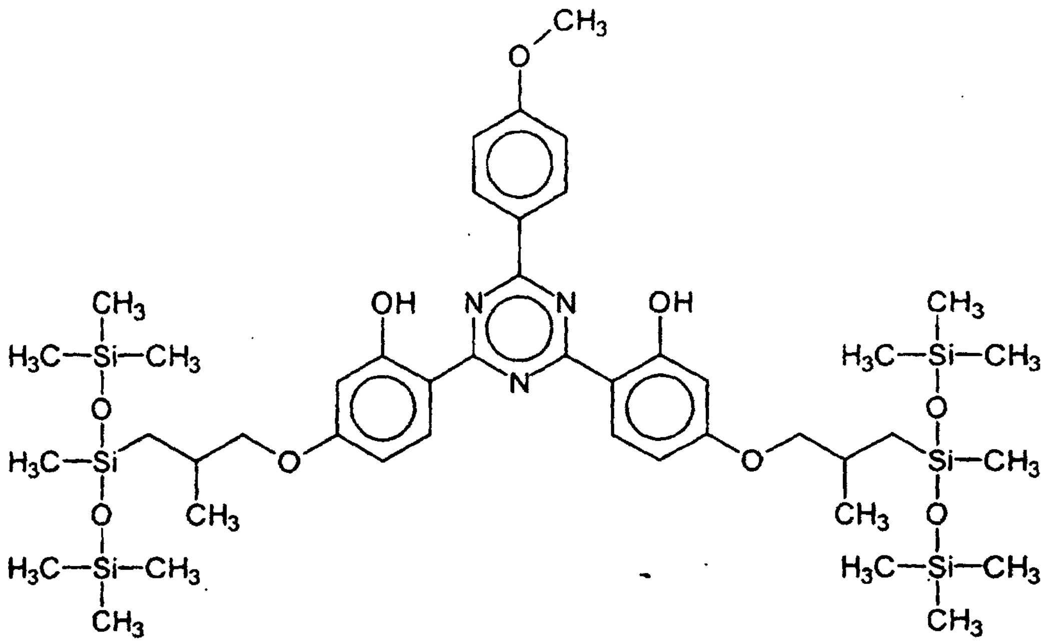

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) mit Bis-Resorcinyltriazinderivaten erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Noch ein weiterer vorteilhafter UV-Filter ist das 2,2'-Methylen-bis-(6-(2H-benzotriazol-2-yl)-4-(1,1,3,3-tetramethylbutyl)-phenol), welches durch die chemische Strukturformel

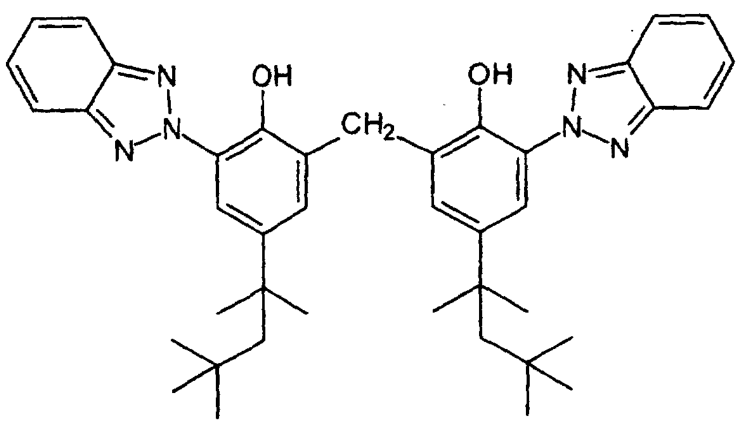

gekennzeichnet ist. Dennoch ergeben sich auch bei dieser Substanz gewisse, formulierungstechnisch bedingte Nachteile bei Einsatz hoher Mengen, denn diese Substanz ist schwerlöslich. Es ist unter Verwendung dieser Substanz schwierig, hohe Lichtschutzfaktoren zu erreichen, da, wie bei anorganischen Mikropigmenten auch, die Wirksamkeit durch die Einarbeitbarkeit in mehrphasige Emulsionssysteme begrenzt wird.

Der Lichtschutzfaktor (LSF, oft auch, dem englischen Sprachgebrauch angepaßt, SPF genannt) gibt an, um wieviel länger die mit dem Lichtschutzmittel geschützte Haut bestrahlt werden kann, bis die gleiche Erythemreaktion auftritt wie bei der ungeschützten Haut (also zehnmal solang gegenüber ungeschützter Haut bei LSF = 10).

Jedenfalls erwartet der Verbraucher zum einen - nicht zuletzt wegen der ins Licht der Öffentlichkeit gerückten Diskussion über das sogenannte "Ozonloch" zum einen zuverlässige Angaben des Herstellers zum Lichtschutzfaktor,

zum anderen geht eine Tendenz des Verbrauchers zu höheren und hohen Lichtschutzfaktoren.

Da Lichtschutzfiltersubstanzen in der Regel kostspielig sind, und da manche Lichtschutzfiltersubstanzen zudem schwierig in höheren Konzentrationen in kosmetische oder dermatologische Zubereitungen einzuarbeiten sind, war es eine weitere Aufgabe der Erfindung, auf einfache und preiswerte Weise zu Zubereitungen zu gelangen, welche bei ungewöhnlich niedrigen Konzentrationen an herkömmlichen Lichtschutzfiltersubstanzen dennoch akzeptable oder sogar hohe LSF-Werte erreichen.

Insbesondere emulgatorfreie Formulierungen (wie z.B. WO-A-98 42300), die für die empfindliche Haut angeboten werden, haben den Nachteil, daß hier hohe Lichtschutzwirkungen besonders schwer einzustellen sind. Bei gleichem LSF zeichnen sich emulgatorfreie Zubereitungen in der Regel durch eine erhöhte Komzentration an UV-Filtern gegenüber echten Emulsionen aus.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, emulgatorfreie Sonnenschutzprodukte zu entwikkeln, welche sich durch hohen Lichtschutzfaktors bzw. UV-A-Schutzleistung auszeichnen.

Emulgatorfreie Lichtschutzpräparate auf Basis sogenannter Hydrodispersionen sind seit einiger Zeit für den Verbraucher zugängig. Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Obwohl dieser Formulierungstyp gegenüber den herkömmlichen Lichtschutzformulierungen den Vorteil der Emulgatorfreiheit bietet, gibt es andererseits auch einige Punkte, welche der Verbesserung bedürfen. So ist für eine gute Lichtschutzwirkung solcher Präparate eine vergleichsweise hohe Konzentration an UV-Filtern notwendig. Darüberhinaus fühlen sich solche Präparate im Vergleich zu Emulsionen und Lichtschutzölen klebrig an.

Eine weitere Aufgabe war daher, Lichtschutzpräparate zur Verfügung zu stellen, welche sich durch eine relativ niedrige Konzentration an UV-Filtern auszeichnen und darüberhinaus ein angenehmes Hautgefühl vermitteln. Eine andere Aufgabe der vorliegenden Erfindung bestand darin, nichtklebrige Lichtschutzpräparate zur Verfügung zu stellen.

Es war überraschend und für den Fachmann nicht vorauszusehen, daß kosmetische und dermatologische Lichtschutzformulierungen in Form von Hydrodispersionen,

(a) welche aus einer inneren Lipidphase und einer äußeren wäßrigen Phase bestehen, und
(b) welche höchstens 0,5 Gew.-% an einem oder mehreren Emulgatoren sind und bevorzugt gänzlich frei von Emulgatoren sind
dadurch gekennzeichnet, daß sie
(c) einen Gehalt an $C_{18\text{-}36}$-Triglyceride aufweisen und
(d) einen Gehalt an Hydrokolloiden aufweisen.

die Lösung dieser Aufgaben darstellen.

Ferner war überraschend, daß die Verwendung von Glyceridwachsen zur Erhöhung der des Lichtschutzfaktors und/oder der UV-A-Schutzleistung kosmetischer oder dermatologischer Zubereitungen, enthaltend mindestens eine übliche UV-A-Filtersubstanz und/oder eine Breitbandfiltersubstanz, die auch gegen UV-Strahlung mit einer Wellenlänge, die größer als 335 nm ist, Schutz gewährt, den Nachteilen des Standes der Technik abhelfen würde.

Die Verwendung von Glyceridwachsen in kosmetischen, dermatologischen oder pharmazeutischen Formulierungen ist an sich bekannt.

Nach Festlegung der Deutschen Gesellschaft für Fettwirtschaft *(Fette, Seifen, Anstrichmittel, **76**, 135 [1974])* werden zur Kennzeichnung des Begriffes "Wachs" in der Regel die mechanisch-physikalischen Eigenschaften der Wachse, welche für ihre Verwendung maßgebend sind, herangezogen, während für die Begriffsbestimmung die jeweilige chemische Zusammensetzung unberücksichtigt bleibt.

"Wachs" ist - ähnlich wie "Harz" - eine Sammelbezeichnung für eine Reihe natürlicher oder künstlich gewonnener Stoffe, die in der Regel folgende Eigenschaften aufweisen: bei 20 °C knetbar, fest bis brüchig hart, grob- bis

feinkristallin, durchscheinend bis opak, jedoch nicht glasartig, über 40 °C ohne Zersetzung schmelzend, schon wenig oberhalb des Schmelzpunkts verhältnismäßig niedrigviskos und nicht fadenziehend, stark temperaturabhängige Konsistenz und Löslichkeit und unter leichtem Druck polierbar. Ist in Grenzfällen bei einem Stoff mehr als eine der vorstehend genannten Eigenschaften nicht erfüllt, so ist er kein Wachs im Sinne dieser Definition. Wachse unterscheiden sich von ähnlichen synthetischen oder natürlichen Produkten (z. B. Harzen, plastischen Massen usw.) hauptsächlich darin, daß sie in der Regel etwa zwischen 50 und 90 °C, in Ausnahmefällen auch bis zu etwa 200 °C, in den schmelzflüssigen, niedrigviskosen Zustand übergehen und praktisch frei von aschebildenden Verbindungen sind.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% Glyceridwachse. "Hydrokolloid" ist die technologische Kurzbezeichnung für die an sich richtigere Bezeichnung hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Die Gruppe der Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

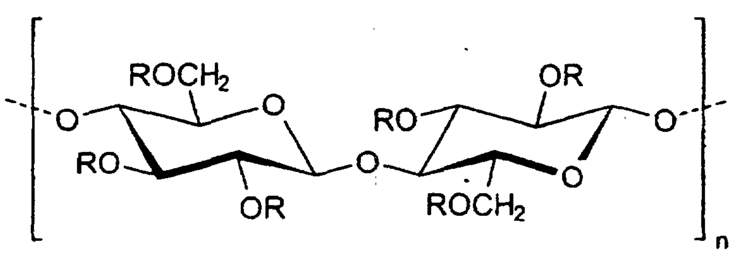

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat.

Erfindungsgemäß vorteilhaft sind ferner Acrylat-Alkylacrylat-Copolymere, insbesondere solche, die aus der Gruppe der sogenannten Carbomere oder Carbopole (Carbopol® ist eigentlich eine eingetragene Marke der B. F.

Goodrich Company) gewählt werden. Insbesondere zeichnen sich das oder die erfindungsgemäß vorteilhaften Acrylat-Alkylacrylat-Copolymere durch die folgende Struktur aus:

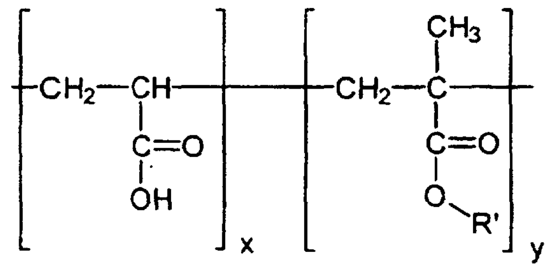

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Alkylacrylat-Copolymere, welche unter den Handelbezeichnungen Carbopol® 1382, Carbopol® 981 und Carbopol® 5984 von der B. F. Goodrich Company erhältlich sind.

Ferner vorteilhaft sind Copolymere aus $C_{10\text{-}30}$-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit. Die INCI-Bezeichnung für solche Verbindungen ist Acrylates/C 10-30 Alkyl Acrylate Crosspolymer". Insbesondere vorteilhaft sind die unter den Handelbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen Hydrodispersionen vorteilhaft kleiner als 1,0 Gew.-%, bevorzugt zwischen 0,01 und 0,1 Gew.-%, bezogen auf das Gesamtgewicht der Zubereitungen, gewählt.

Es war erstaunlich, daß die Glyceridwachse enthaltenden Hydrodispersioneri im Sinne der vorliegenden Erfindung zu einer Steigerung des Lichtschutzfaktors und/oder der UV-A-Schutzleistung führen würden, da diese Glyceridwachse selbst über keine ausgeprägte Absorption im UVA- oder UVB-Bereich verfügen.

Insbesondere war erstaunlich, daß die Glyceridwachse enthaltenden Hydrodispersionen im Sinne der vorliegenden Erfindung zu einer Steigerung des Lichtschutzfaktors und/oder der UV-A-Schutzleistung führen würden, wenn wenigstens einer der UV-Filter in den Lichtschutzzubereitungen gemäß der vorliegenden Erfindung aus der Gruppe der Triazinderivate gewählt wird, insbesondere gewählt aus der Gruppe

- 4,4',4"-(1,3,5-Triazin-2,4,6-triyltriimino)-tris-benzoesäure-tris(2-ethylhexylester)
- 2,4-Bis-{[4-(2-Ethyl-hexyloxy)-2-hydroxy]-phenyl}-6-(4-methoxyphenyl)-1,3,5-triazin,
- 2,4-Bis-{[4-(3-sulfonato)-2-hydroxy-propyloxy)-2-hydroxy]-phenyl}-6-(4-methoxyphenyl)-1,3,5-triazin Natriumsalz,
- 2,4-Bis-{[4-(3-(2-Propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin,
- 2,4-Bis-{[4-(2-ethyl-hexyloxy)-2-hydroxy]-phenyl}-6-(1-methy)-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-{[4-(2"-methylpropenyloxy)-2-hydroxy]-phenyl}-6-(4-methoxyphenyl)-1,3,5-triazin,
- 2,4-Bis-{[4-(1',1',1',3',5',5',5'-Heptamethylsiloxy-2"-methyl-propyloxy)-2-hydroxy]phenyl}-6-(4-methoxyphenyl)-1,3,5-triazin.

Ferner war erstaunlich, daß die Glyceridwachse enthaltenden Hydrodispersionenim Sinne der vorliegenden Erfindung zu einer Steigerung des Lichtschutzfaktors und/oder der UV-A-Schutzleistung führen würden, wenn wenigstens einer der UV-Filter in den Lichtschutzzubereitungen gemäß der vorliegenden Erfindung das 2,2'-Methylen-bis-(6-(2H-benzotriazol-2-yl)-4-(1,1,3,3-tetramethylbutyl)-phenol) ist.

Vorteilhafte UV-A-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter sind beispielsweise Bis-Resorcinyltriazinderivate mit der folgenden Struktur:

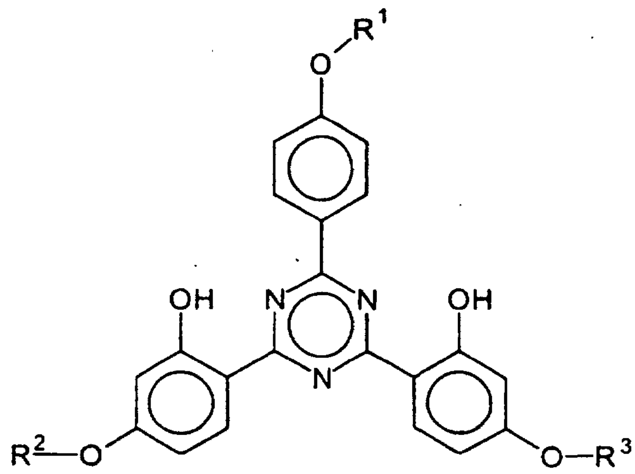

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethyl-hexyloxy)-2-hydroxy]-phenyl}-6-(4-methoxyphenyl)-1,3,5-triazin, welches durch folgende Struktur gekennzeichnet ist:

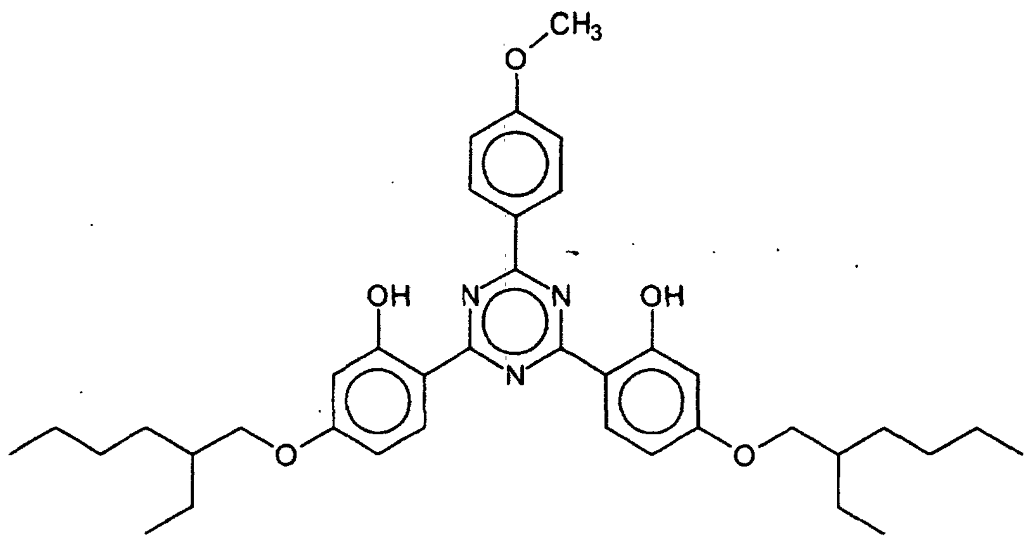

Kosmetische und dermatologische Formulierungen im Sinne der Erfindung enthalten vorteilhaft einen oder mehrere übliche UV-A-Filter als Einzelsubstanzen oder in beliebigen Gemischen untereinander, in der Lipidphase und/ oder in der wäßrigen Phase.

Besonders bevorzugt ist die Verwendung von $C_{18-36}$-Triglyceriden zur Erhöhung des Lichtschutzfaktors und/ oder der UV-A-Schutzleistung von Dibenzoylmethanderivaten, insbesondere von 5-Isopropyldibenzoylmethan und/ oder 4-(tert.-Butyl)-4'-methoxydibenzoylmethan. Erstaunlicherweise steigert die Verwendung von $C_{18-36}$-Triglyceriden die UV-A-Schutzleistung von Dibenzoylmethanderivaten um mehr als 75 %.

Die Gesamtmenge an UV-A-Filtersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Kosmetische und dermatologische Zubereitungen gemäß der Erfindung enthalten vorteilhaft, wenngleich nicht zwingend, anorganische Pigmente, welche röntgenamorph oder nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Alumi-

niums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich. Vorteilhafte $TiO_2/Fe_2O_3$-Mischoxide sind beispielsweise unter der Handelsbezeichnung T 817 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-,

Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoëharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase der erfindungsgemäßen Hydrodispersionen kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase derHydrodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der flüssigen Fettsäuretriglyceride. Die flüssigen Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat

und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß werden der Lichtschutzfaktor und/oder die UV-A-Schutzleistung durch die Verwendung von Glyceridwachsen in Hydrodispersionen, enthaltend übliche UVA-Filter und/oder UVB-Filter in der Lipidphase und/oder in der wäßrigen Phase erheblich gesteigert.

Insbesondere werden der Lichtschutzfaktor und/oder die UV-A-Schutzleistung durch die Verwendung von Glyceridwachsen in Hydrodispersionen, enthaltend Substanzen, die UV-Strahlung im UVB-Bereich absorbieren, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Solche UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Insbesondere werden der Lichtschutzfaktor und/oder die UV-A-Schutzleistung durch die Verwendung von Glyceridwachsen in Hydrodispersionen, enthaltend Substanzen, die UV-Strahlung im UVA-Bereich absorbieren, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, erheblich gesteigert.

Demgemäß ist vorteilhaft auch auch die erfindungsgemäße Verwendung, dadurch gekennzeichnet, daß die Hydrodispersionen als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen-1,4-bis-(2-benzimidazyl)-3,3'-5,5'-tetrasulfonsäure-bis-natriumsalz, enthalten.

Es kann auch von erheblichem Vorteil sein, polymergebundene oder polymere UV-Filtersubstanzen in Hydrodispersionen gemäß der vorliegenden Erfindung zu verwenden, insbesondere solche, wie sie in der WO-A-92/20690 beschrieben werden.

Insbesondere werden der Lichtschutzfaktor und/oder die UV-A-Schutzleistung durch die Verwendung von Glyceridwachsen in Hydrodispersionen, enthaltend polymergebundene oder polymere UV-Filtersubstanzen, wobei die Gesamtmenge solcher Filtersubstanzen z.B. 0,1 Gew.-% bis 30 Gew.-%, vorzugsweise 0,5 bis 20 Gew.-%, insbesondere 3 bis 15 Gew.-% beträgt, bezogen auf das Gesamtgewicht der Zubereitungen, erheblich gesteigert.

Die Gesamtmenge an wasserlöslichen UV-Filtersubstanzen in den fertigen Hydrodispersionen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Phenylbenzimidazol-5-sulfonsäure bzw. deren Salzen in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Hydroxy-4-methoxybenzophenon-5-sulfonsäurebzw. deren Salzen in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(2-Oxo-3-bornylidenmethyl)benzolsulfonsäure bzw. deren Salzen in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Methyl-5-(2-oxo-3-bornylidenmethyl)benzolsulfonsäur bzw. deren Salzen in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an Benzol-1,4-di(2-oxo-3-bornylidenmethyl-10-Sulfonsäurebzw. deren Salzen in den fertigen Hydrodispersionen wird; falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4,4',4"-(1,3,5-Triazin-2,4,6-triyltriimino)-tris-benzoesäure-tris(2-ethylhexylester) in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-(tert.-Butyl)-4'-methoxydibenzoylmethan in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 4-Methylbenzylidencampher in den fertigen Hydrodispersionen wird, falls die Gegenwart dieser Substanz erwünscht ist, vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an 2-Ethylhexyl-p-methoxy-cinnamat in den fertigen Hydrodispersionen wird, falls die Gegenwart dieser Substanz erwünscht ist, vorteilhaft aus dem Bereich von 0,1 - 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

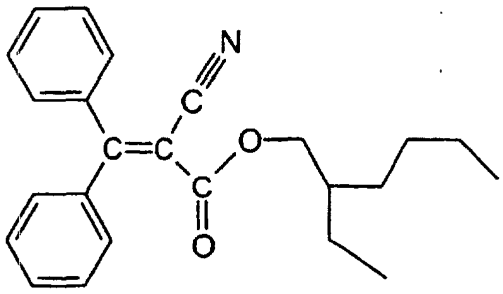

Die Gesamtmenge an Ethylhexyl-2-cyano-3,3-diphenylacrylat in den fertigen Hydrodispersionen wird, falls die Gegenwart dieser Substanz erwünscht ist, vorteilhaft aus dem Bereich von 0,1 - 15,0 Gew.-%, bevorzugt 0,5 - 10,0

Gew.-% gewählt, bezogen auf das Gesamtgewicht der Zubereitungen.

Ferner kann gegebenenfalls von Vorteil sein, erfindungsgemäß weitere UVA- und/oder UVB-Filtem in Hydrodispersionen einzuarbeiten, beispielsweise bestimmten Salicylsäurederivaten wie 4-Isopropylbenzylsalicylat, 2-Ethylhexylsalicylat (= Octylsalicylat), Homomenthylsalicylat.

Die Gesamtmenge an einem oder mehreren Salicylsäurederivaten in den fertigen Hydrodispersionen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Der Lichtschutzfaktor und/oder die UV-A-Schutzleistung von Hydrodispersionen, enthaltend beiliebige der vorab geschilderten UV-Filtersubstanzen, sei es als Einzelsubstanzen oder in beliebigen Gemischen untereinander, wobei die Gesamtmenge solcher Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, werden erfinclungsgemäß durch die Verwendung von Glyceridwachsen erheblich gesteigert.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Beispiel 1

| | Gew.-% |
|---|---|
| Caprylsäure/Caprinsäuretriglycerid | 10,00 |
| Dicaprylylether | 5,00 |
| Dimethicone | 2,00 |
| Hydriertes Polyisobuten | 2,00 |
| Vitamin E-Acetat | 0,50 |
| Octyltriazon | 2,00 |
| Dioctylbutylamidotriazon | 2,00 |
| Butyl Methoxydibenzoyl Methan | 2,00 |
| Titandioxid | 1,00 |
| C18-36 Triglycerid | 5,00 |
| Glycerin | 3,00 |
| Xanthan Gummi | 0,50 |
| Carbopol 5984 | 0,10 |
| Natronlauge 45% | 0,10 |
| Konservierungsmittel, Parfum, Farbstoffe | q.s. |
| Wasser | ad 100,00 |

Beispiel 2

| | Gew.-% |
|---|---|
| Ricinusöl | 2,00 |
| Butylenglycoldicaprylat/Caproat | 10,00 |
| $C_{12-15}$ -Alkylbenzoat | 5,00 |
| Octyltriazon | 4,00 |
| Methylbenzylidencampher | 4,00 |
| Butylmethoxydibenzoylmethan | 2,00 |
| $C_{18-36}$-Triglycerid | 2,00 |
| $C_{16-24}$-Triglycerid | 2,00 |
| Glycerin | 10,00 |

(fortgesetzt)

| | Gew.-% |
|---|---|
| Xanthan Gummi | 0,20 |
| Pemulen TR1 | 0,50 |
| Phenylbenzimidazol sulfonsäure | 2,00 |
| Natronlauge 45% | 1.20 |
| Konservierungsmittel, Parfum, Farbstoffe | q.s. |
| Wasser | ad 100,00 |

Beispiel 3

| | Gew.-% |
|---|---|
| Dimethicone | 5,00 |
| Mineralöl | 2,00 |
| Isohexadekan | 2,00 |
| Hydriertes Polyisobuten | 5,00 |
| Butylenglycoldicaprylat/Caproat | 5,00 |
| Octyltriazon | 2,00 |
| Methylbenzyliden Campher | 2,00 |
| Octylmethoxycinnamat | 5,00 |
| $C_{18-36}$-Triglycerid | 3,00 |
| Glycerin | 5,00 |
| Hydroxypropymethylcellulose | 0,50 |
| Hectorit | 1,00 |
| Konservierungsmittel, Parfum, Farbstoffe | q.s. |
| Wasser | ad 100,00 |

Beispiel 4

| | Gew.-% |
|---|---|
| Caprylsäure/Caprinsäuretriglycerid | 5,00 |
| Octyldodecanol | 5,00 |
| Butylenglycoldicaprylat/Caproat | 5,00 |
| Octyltriazon | 4,00 |
| Dioctylbutylamidotriazon | 4,00 |
| Methylbenzylidencampher | 4,00 |
| Butylmethoxydibenzoylmethan | 4,00 |
| $TiO_2$ | 2,00 |
| $C_{18-36}$-Triglycerid | 3,00 |
| $C_{16-24}$-Triglycerid | 1,00 |
| Glycerin | 5,00 |
| Xanthan Gummi | 0,20 |
| Hydroxypropymethylcellulose | 0,20 |
| Phenylbenzimidazolsulfonsäure | 4,00 |
| Natronlauge 45 % | 1,30 |
| Wasser | ad 100,00 |

## Patentansprüche

1. Kosmetische und nicht-therapeutisch dermatologische Lichtschutzformulierungen in Form von Hydrodispersionen,

   - (a) welche aus einer inneren Lipidphase und einer äußeren wäßrigen Phase bestehen, und
   (b) welche höchstens 0,5 Gew.-% an einem oder mehreren Emulgatoren sind und bevorzugt gänzlich frei von Emulgatoren sind,
   **dadurch gekennzeichnet, daß** sie
   (c) einen Gehalt an Glyceridwachsen aufweisen, welche aus der Gruppe der $C_{18-36}$ -Triglyceride gewählt werden und
   (d) einen Gehalt an Hydrokolloiden aufweisen.

2. Lichtschutzformulierungen nach Anspruch 1, **dadurch gekennzeichnet, daß** sie 0,1 bis 20 Gew.-% Glyceridwachse enthalten.

3. Lichtschutzformulierungen nach Anspruch 1, **dadurch gekennzeichnet, daß** sie 0,5 bis 10 Gew.-% Glyceridwachse enthalten.

4. Lichtschutzformulierungen nach Anspruch 1, **dadurch gekennzeichnet, daß** sie 1 bis 5 Gew.-% Glyceridwachse enthalten.

5. Lichtschutzformulierungen nach Anspruch 1, **dadurch gekennzeichnet, daß** als UV-Filtersubstanzen Triazinderivate gewählt werden aus der Gruppe

   - 4,4',4"-(1,3,5-Triazin-2,4,6-triyltriimino)-tris-benzoesäure-tris(2-ethylhexytester)
   - 2,4-Bis-{[4-(2-Ethyl-hexyloxy)-2-hydroxy]-phenyl}-6-(4-methoxyphenyl)-1,3,5-triazin,
   - 2,4-Bis-{[4-(3-sulfonato)-2-hydroxy-propyloxy)-2-hydroxy]-phenyl}-6-(4-methoxyphenyl)-1,3,5-triazin Natriumsalz,
   - 2,4-Bis-{[4-(3-(2-Propyloxy)-2-hydroxy-propyloxy]-2-hydroxy]-phenyl}-6-(4-methoxyphenyl}-1,3,5-triazin,
   - 2,4-Bis-{[4-(2-ethyl-hexyloxy)-2-hydroxy]-phenyl}-6-[4-(2-methoxyethylcarboxyl)-phenylamino)-1,3,5-triazin,
   - 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin,
   - 2,4-Bis-{[4-(2-ethyl-hexyloxy)-2-hydroxy]-phenyl}-6-(9-methyl-pyrrol-2-yl)-1,3,5-triazin,
   - 2,4-Bis-{[4-tris(trimethylsiloxy-silylpropyloxy)-2-hydroxy]-phenyl}-6-(4-methoxyphenyl)-1,3,5-triazin,
   - 2,4-Bis-{[4-(2"-methylpropenyloxy)-2-hydroxy]-phenyl}-6-(4-methoxyphenyl)-1,3,5-triazin,
   - 2,4-Bis-{[4-(1',1',1',3',5,5',5'-Heptamethylsiloxy-2"-methyl-propyloxy)-2-hydroxy]-phenyl}-6-(4-methoxyphenyl)1,3,5-triazin.

6. Lichtschutzformulierungen nach Anspruch 1, **dadurch gekennzeichnet, daß** sie als UV-A-Filtersubstanz ein oder mehrere fettlösliche UV-A-Filtersubstanzen, besonders Dibenzoylmethanderivate, insbesondere 5-Isopropyldibenzoylmethan und/oder 4-(tert.-Buty))-4'-methoxydibenzoylmethan, enthalten.

7. Lichtschutzformulierungen nach Anspruch 1, **dadurch gekennzeichnet, daß** sie als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen-1,4-bis-(2-benzimidazyl)-3,3'-5,5'-tetrasulfonsäure-bis-natriumsalz, enthalten.

8. Lichtschutzformulierungen nach Anspruch 1, enthaltend ein oder mehrere Glyceridwachse sowie 2,2'-Methylen-bis-(6-(2H-benzotriazol-2-yl)-4-(1,1,3,3-tetramethylbutyl)-phenol).

9. Lichtschutzformulierungen nach Anspruch 1, enthaltend eine Gesamtmenge an einem oder mehreren Hydrokolloiden von weniger als 1,0 Gew.-%, bevorzugt zwischen 0,01 und 0,1 Gew.-%, bezogen auf das Gesamtgewicht der Zubereitungen.

## Claims

1. Cosmetic and non-therapeutic dermatological photoprotective formulations in the form of hydrodispersions

   (a) which consist of an inner lipid phase and an outer aqueous phase, and
   (b) which are at most 0.5% by weight of one or more emulsifiers and are preferably entirely free from emulsifiers, **characterized in that** they
   (c) have a content of glyceride waxes which are chosen from the group $C_{18-36}$-triglycerides and
   (d) have a content of hydrocolloids.

2. Photoprotective formulations according to Claim 1, **characterized in that** they comprise 0.1 to 20% by weight of glyceride waxes.

3. Photoprotective formulations according to Claim 1, **characterized in that** they comprise 0.5 to 10% by weight of glyceride waxes.

4. Photoprotective formulations according to Claim 1, **characterized in that** they comprise 1 to 5% by weight of glyceride waxes.

5. Photoprotective formulations according to Claim 1, **characterized in that** the UV filter substances are triazine derivatives chosen from the group consisting of

   - tris(2-ethylhexyl) 4,4',4"-(1,3,5-triazine-2,4,6-triyltriimino)trisbenzoate
   - 2,4-bis{[4-(2-ethylhexyloxy)-2-hydroxy]phenyl}-6-(4-methoxyphenyl)-1,3,5-triazine,
   - 2,4-bis{[4-(3-sulphonato)-2-hydroxypropyloxy)-2-hydroxy]phenyl}-6-(4-methoxyphenyl)-1,3,5-triazine sodium salt,
   - 2,4-bis{[4-(3-(2-propyloxy)-2-hydroxypropyloxy)-2-hydroxy]phenyl}-6-(4-methoxyphenyl)-1,3,5-triazine,
   - 2,4-bis{[4-(2-ethylhexyloxy)-2-hydroxy]phenyl}-6-[4-(2-methoxyethylcarboxyl)phenylamino]-1,3,5-triazine,
   - 2,4-bis{[4-(3-(2-propyloxy)-2-hydroxypropyloxy)-2-hydroxy]phenyl}-6-[4-(2-ethylcarboxyl)-phenylamino]-1,3,5-triazine,
   - 2,4-bis{[4-(2-ethylhexyloxy)-2-hydroxy]phenyl}-6-(1-methylpyrrol-2-yl)-1,3,5-triazine,
   - 2,4-bis{[4-tris(trimethylsiloxysilylpropyloxy)-2-hydroxy]phenyl}-6-(4-methoxyphenyl)-1,3,5-triazine,
   - 2,4-bis{[4-(2"-methylpropenyloxy)-2-hydroxy]-phenyl}-6-(4-methoxyphenyl)-1,3,5-triazine,
   - 2,4-bis{[4-(1',1',1',3',5',5',5'-heptamethylsiloxy-2"-methylpropyloxy)-2-hydroxy]phenyl}-6-(4-methoxyphenyl)-1,3,5-triazine.

6. Photoprotective formulations according to Claim 1, **characterized in that** they comprise, as UV-A filter substance, one or more fat-soluble UV-A filter substances, particularly dibenzoylmethane derivatives, in particular 5-isopropyldibenzoylmethane and/or 4-(tert-butyl)-4'-methoxydibenzoylmethane.

7. Photoprotective formulations according to Claim 1, **characterized in that** they comprise, as UV-A filter substance, one or more water-soluble UV-A filter substances, particularly water-soluble UV-A filter substances chosen from the group consisting of phenylene-1,4-bis(2-benzimidazyl)-3,3'-5,5'-tetrasulphonic acid and/or 1,4-di(2-oxo-10-sulpho-3-bornylidenemethyl)benzene and/or its salts, particularly the corresponding sodium, potassium or triethanolammonium salts and/or the corresponding 10-sulphato compounds, in particular the phenylene-1,4-bis(2-benzimidazyl)-3,3'-5,5'-tetrasulphonic acid bis-sodium salt.

8. Photoprotective formulations according to Claim 1, comprising one or more glyceride waxes, and 2,2'-methylenebis (6-(2H-benzotriazol-2-yl)-4-(1,1,3,3-tetramethylbutyl)phenol).

9. Photoprotective formulations according to Claim 1, comprising a total amount of one or more hydrocolloids of less than 1.0% by weight, preferably between 0.01 and 0.1% by weight, based on the total weight of the preparations.

## Revendications

1. Compositions photoprotectrices cosmétiques et dermatologiques non thérapeutiques, sous forme d'hydrodispersions,

(a) qui consistent en une phase lipidique interne et une phase aqueuse externe et
(b) qui ont une teneur d'au maximum 0,5 % en poids en un ou plusieurs émulsifiants et de préférence sont totalement exemptes d'émulsifiants,
**caractérisées en ce qu'**elles
(c) présentent une teneur en cires de glycérides qui sont choisies dans le groupe des triglycérides en $C_{18-36}$ et
(d) présentent une teneur en hydrocolloïdes.

**2.** Compositions photoprotectrices selon la revendication 1, **caractérisées en ce qu'**elles contiennent 0,1 à 20 % en poids de cires de glycérides.

**3.** Compositions photoprotectrices selon la revendication 1, **caractérisées en ce qu'**elles contiennent 0,5 à 10 % en poids de cires de glycérides.

**4.** Compositions photoprotectrices selon la revendication 1, **caractérisées en ce qu'**elles contiennent 1 à 5 % en poids de cires de glycérides.

**5.** Compositions photoprotectrices selon la revendication 1, **caractérisées en ce qu'**en tant que substances filtrant les UV sont choisis des dérivés de triazine dans le groupe constitué par les dérivés suivants

- 4,4',4''-(1,3,5-triazine-2,4,6-triyltri-imino)-trisbenzoate de tris(2-éthylhexyle),
- 2,4-bis-{[4-(2-éthyl-hexyloxy)-2-hydroxy]-phényl}-6-(4-méthoxyphényl)-1,3,5-triazine,
- sel de sodium de 2,4-bis-{[4-(3-sulfonato)-2-hydroxy-propyloxy)-2-hydroxy]-phényl}-6-(4-méthoxyphényl)-1,3,5-triazine,
- 2,4-bis-{[4-(3-(2-propyloxy)-2-hydroxy-propyloxy)-2-hydroxy]-phényl}-6-(4-méthoxyphényl)-1,3,5-triazine,
- 2,4-bis-{[4-(2-éthyl-hexyloxy)-2-hydroxy]-phényl}-6-[4-(2-méthoxyéthyl-carboxy)-phénylamino]-1,3,5-triazine,
- 2,4-bis-{[4-(3-(2-propyloxy)-2-hydroxy-propyloxy)-2-hydroxy]-phényl}-6-[4-(2-éthylcarboxy)-phénylamino]-1,3,5-triazine,
- 2,4-bis-{[4-(2-éthyl-hexyloxy)-2-hydroxy]-phényl}-6-(1-méthyl-pyrrol-2-yl)-1,3,5-triazine,
- 2,4-bis-{[4-tris(triméthylsiloxy-silylpropyloxy)-2-hydroxy]-phényl}-6-(4-méthoxyphényl)-1,3,5-triazine,
- 2,4-bis-{(4-(2''-méthylpropényloxy)-2-hydroxy]-phényl}-6-(4-méthoxyphényl)-1,3,5-triazine,
- 2,4-bis-{[4-(1',1',1',3',5',5',5'-heptaméthylsiloxy-2''-méthyl-propyloxy)-2-hydroxy]-phényl}-6-(4-méthoxyphényl)-1,3,5-triazine.

**6.** Compositions photoprotectrices selon la revendication 1, **caractérisées en ce qu'**elles contiennent en tant que substance filtrant les UV-A une ou plusieurs substances liposolubles filtrant les UV-A, en particulier des dérivés de dibenzoylméthane, notamment le 5-isopropyldibenzoylméthane et/ou le 4-(tert-butyl)-4'-méthoxydibenzoylméthane.

**7.** Compositions photoprotectrices selon la revendication 1, **caractérisées en ce qu'**elles contiennent en tant que substance filtrant les UV-A une ou plusieurs substances hydrosolubles filtrant les UV-A, en particulier une ou plusieurs substances hydrosolubles filtrant les UV-A choisies dans le groupe constitué par l'acide phénylène-1,4-bis(2-benzimidazyl)-3,3'-5,5'-tétrasulfonique et/ou le 1,4-di(2-oxo-10-sulfo-3-bornylidèneméthyl)benzène et/ou leurs sels, en particulier les sels de sodium, potassium ou triéthanolammonium correspondants et/ou les composés 10-sulfato correspondants, notamment le phénylène-1,4-bis-(2-benzimidazyl)-3,3'-5,5'-tétrasulfonate disodique.

**8.** Compositions photoprotectrices selon la revendication 1, contenant une ou plusieurs cires de glycérides ainsi que du 2,2-méthylène-bis(6-2H-benzotriazol-2-yl)-4-(1,1,3,3-tétraméthylbutyl)phénol.

**9.** Compositions photoprotectrices selon la revendication 1, contenant une quantité totale d'un ou plusieurs hydrocolloïdes de moins de 1,0 % en poids; de préférence entre 0,01 et 0,1 % en poids, par rapport au poids total des préparations.